Europäisches Patentamt

European Patent Office

Office européen des brevets

(11) Veröffentlichungsnummer: **0 392 041**
**A1**

## (12) EUROPÄISCHE PATENTANMELDUNG

(21) Anmeldenummer: 89106408.1

(22) Anmeldetag: 11.04.89

(51) Int. Cl.5: **C02F 3/12, C02F 3/04, C02F 3/24**

(43) Veröffentlichungstag der Anmeldung:
17.10.90 Patentblatt 90/42

(84) Benannte Vertragsstaaten:
**BE CH DE FR LI NL**

(71) Anmelder: **VEG (T) RIPPERSHAUSEN**

**DDR-6101 Rippershausen(DE)**

(72) Erfinder: **Beyer, Gerhard, Prof. Dr. Ing.
Hardtstrasse 22
DDR-6206 Dorndorf/Rh.(DD)**
Erfinder: **Strauch, Hellmuth
Marktplatz 1a
DDR-6101 Oberkatz(DD)**
Erfinder: **Abt, Wilmar, Dipl. agr. Ing. ök.
Marktplatz 11a
DDR-6101 Oberkatz(DD)**

(74) Vertreter: **Patentanwälte Beetz sen. - Beetz
jun. Timpe - Siegfried - Schmitt-Fumian
Steinsdorfstrasse 10
D-8000 München 22(DE)**

(54) **Verfahren und Einrichtung zur Aufbereitung von Gülle oder Abwasser.**

(57) Die Erfindung betrifft ein Verfahren zur Durchführung des Stoffaustausches bei der Aufbereitung von Gülle oder Abwasser, die in einer Biogasanlage behandelt wurden, bei dem die Gülle in einem Oxidationsvorgang partiell einer Gegenstrombelüftung ausgesetzt und dann einer Kammer zugeführt wird, das durch folgende wesentliche Verfahrensschritte gekennzeichnet ist:

a) von drei zusammengeschalteten Kammern (8;8';8") mit jeweils zugeordnetem Stoffaustauschapparat in Form eines Klein-Kühlturms (3) wird die erste Kammer bis zu einem Zehntel des Kammervolumens gefüllt;

b) Oxidation der in der ersten Kammer vorhandenen und der weiter zulaufenden Gülle über den Stoffaustauschapparat, mit dem die Gülle versprüht und in einem Rieselfilm wieder vereinigt, belüftet, in eine Auffangwanne abgerieselt, weiterbelüftet und der Kammer wieder zugeführt wird;

c) nach Füllung der Kammer weitere Oxidation der Gülle über den Stoffaustauschapparat bis zu ihrer vollständigen Absättigung;

d) Sedimentation bei gleichzeitiger Oxidation;

e) Entleerung der Kammer nach der Sedimentation und Abschluß der Oxidation;

f) phasenversetzte Füllung der weiteren Kammern für eine separate Oxidation mittels des jeweils zugeordneten Stoffaustauschapparates und Vornahme der Verfahrensschritte a bis e;

g) Ablauf der Pahsenverschiebung um jeweils eine Kammerfüllung.

EP 0 392 041 A1

## Verfahren und Einrichtung zur Aufbereitung von Gülle oder Abwasser

Die Erfindung betrifft ein Verfahren zur Durchführung des Stoffaustausches bei der Aufbereitung von Gülle oder Abwasser, die in einer Biogasanlage behandelt wurden, wobei die Gülle einem Oxidationsvorgang, partiell einer Gegenstrombewegung, ausgesetzt und dann einer Kammer zugeführt wird und eine Einrichtung zur Durchführung des Verfahrens.

Bei der Aufbereitung von stickstoffhaltigem, organisch belastetem Abwasser werden die organischen Inhaltsstoffe biochemisch abgebaut und anschließend die Stickstoffverbindungen eliminiert. Entsprechende Aufbereitungsanlagen enthalten in der Regel mehrere Stahl- oder Stahlbetonbecken mit verschiedenen Querschnitten, die durch ein Pumpwerk und ein Rohrsystem miteinander verbunden sind. Die insbesondere bei kleineren Anlagen kompakt ausgeführten Einzelbecken sind in z. B. Nitrifikations- und Belebungsbecken sowie Vorklär- und Nachklärbecken unterteilt. Die Nachklärbecken sind bei Aufbereitungsanlagen der industriellen Tierproduktion in der Regel als Trichterbecken mit rundem Querschnitt ausgeführt. Diese erfordern je nach Durchsatzmenge und Schlammanfall eine Bautiefe, welche die der Anlage erheblich überschreitet und damit eine Vergrößerung der Gesamtanlage bedingt. Aus der kommunalen Abwasseraufbereitung sind zur Nachklärung von Abwasser mit geringen Feststoffanteilen Röhrensedimentationsbecken bekannt, die jedoch bei Einrichtungen der industriellen Tierproduktion aufgrund des hohen Feststoffgehaltes und der zu erwartenden Betriebsstörungen durch Verstopfung des Rohrsystems nicht eingesetzt werden können.

In den Denitrifikations- und Belebungsbecken entstehen beträchtliche Wärmemengen beim Abbau des organischen Substrats, während in der Nitrifikationsstufe, bedingt durch lange Reaktionszeiten, merkliche Wärmeverluste und entsprechend geringe Temperaturen auftreten. Daher können bei Einzelbecken wegen der räumlichen Trennung keine optimalen Prozeßbedingungen erreicht werden.

Bekannte Anlagen haben daher die Nachteile, daß sie aufgrund der spezifischen Bauform und Bautiefe, insbesondere der Nachklärbecken, keine oder eine nur teilweise Kompaktierung ermöglichen. Ferner läßt sich bei dieser Anordnung der Anlagenteile der Wärmehaushalt der Gesamtanlage nicht beeinflussen. Die Anlagen werden daher in ihrem Volumenbedarf nach den zu erwartenden minimalen Reaktionstemperaturen ausgelegt und erfordern somit einen hohen Flächenbedarf sowie einen erheblichen materiellen und finanziellen Aufwand.

Die DD-PS 148 944 zeigt eine Einrichtung zur biochemischen Behandlung flüssiger Abfallstoffe aus einer industriellen Tierproduktion und anderer konzentrierter stickstoffhaltiger Abwässer, die im wesentlichen aus Belebungsbecken, Nachklärbecken, Nitrifikations- und Denitrifikationsbecken, Schlammeindickungsbecken, Pumpwerk und Meßwerte besteht. Die zum Substratabbau und zur mikrobiologischen Stickstoffeliminierung erforderlichen Teilanlagen sowie die notwendigen Nachklär- und Betriebseinrichtungen sind entsprechend dem technologischen Ablauf zellenartig in einem Kompaktbau eingeordnet, wobei die Nachklärbecken als Sedimentationsbecken ausgeführt sind und das Nitrifikationsbecken mindestens von drei Seiten von anderen Becken und Einrichtungen umschlossen ist. Nachteilig ist dabei, daß die Kompaktbauweise nur in einer Aneinanderfügung von mehreren Becken unterschiedlicher Funktion sowie zur Schlammeindickung besteht. Dadurch wird der mit einer Kompaktbauweise beabsichtigte Vorderteil wieder aufgehoben.

Aus der DE-OS 32 44 963 ist weiterhin eine Einrichtung zur Reinigung von Abwasser mittels einer katalytischen Oxidation innerhalb eines Reaktionsbeckens bekannt, wobei das Abwasser dem Becken an einer Stirnwand zugeführt und das Reinwasser an der gegenüberliegenden Stirnwand abgeführt wird. Das Becken ist in mehrere Kammern unterteilt, die jeweils einen Steigkanal mit mehreren Querwänden aufweisen. Die Querwände sind mit einer Pendelklappe versehen oder entgegen der Strömungsrichtung des Wassers geneigt angeordnet, um ein Verschleppen der Katalysatorteilchen zu verhindern. Die Kammern dieser Becken weisen einen Zu-und einen Ablauf auf und dienen ausschließlich zur Anreicherung des Wasser-Kontakt-Masse-Luftgemisches vom Kammerboden aus. Die Lüftungsklappen in den Kammern sollen lediglich einer Zerstörung der Katalysatoren und ihrem Verschleppen entgegenwirken. Der Anschluß der Zu- und Abläufe in den Stirnwänden der Kammern erfolgt nur in einer Höhe und dient dem Abzug der dünnen Stoffe in einer Ebene.

Die DD-PS 94 963 offenbart ein Verfahren und eine Einrichtung zur $CO_2$-Austreibung eines tropfenförmigen Abwassers mittels eines Sieb- und Düsenbodens, der über einer mit einem durchlässigen Trogboden angeordneten Wirbelkammer vorgesehen ist, in der das abtropfende Abwasser einer Gegenstrombelüftung ausgesetzt ist.

Die bekannten Verfahren und Einrichtungen weisen den Nachteil auf, daß eine angestrebte Kompaktbauweise nicht mit der notwendigen Konsequenz einer vielgestalteten Verwendung der Einrichtung für mehrere Verfahrensstufen bei gleichen Einrichtungsteilen vorgenommen werden kann.

Der Erfindung liegt die Aufgabe zugrunde, ein Verfahren zur Durchführung des Stoffaustausches bei der Aufbereitung von Gülle und Abwasser, die in einer Biogasanlage behandelt wurden, aufzuzeigen, das eine optimierte kontinuierliche und stufenförmige Verfahrensführung des Stoffaustausches bis zur Absättigung des BSB und der Nitrifikation in einer Mehrfachfunktion bei gleichzeitiger Realisierung mehrerer in sich geschlossener Verfahrensschritte bei intensiver Verwertung der Prozeßwärme ermöglicht. Ferner soll eine kompakte, raum- und flächensparende Einrichtung geschaffen werden, die bei minimalen Baukosten eine um das Mehrfache gesteigerte Durchsatz- und Reinigungsleistung besitzt.

Erfindungsgemäß wird diese Aufgabe bei dem Verfahren zur Durchführung des Stoffaustausches bei der Aufbereitung von Gülle oder Abwasser dadurch gelöst, daß die Gülle während des Oxidationsvorgangs eine erste Kammer füllend und sich phasenversetzt in weiteren Kammern fortsetzend einer Gegenstrombelüftung ausgesetzt, bei laufender Oxidation mehrfach versprüht und wieder vereinigt, belüftet und bei gleichzeitiger Sedimentation bis zur vollständigen Absättigung der Gülle behandelt wird.

Im einzelnen sind erfindungsgemäß folgende Maßnahmen vorgesehen:

a) Von drei zusammengeschalteten Kammern mit jeweils zugeordnetem Stoffaustauschapparat in Form eines Kleinkühlturms wird die erste Kammer bis zu einem Zehntel des Kammervolumens gefüllt;

b) die Oxidation der in der ersten Kammer vorhandenen und der weiter zulaufenden Gülle erfolgt über einen Stoffaustauschapparat, mit dem die Gülle versprüht und in einem Rieselfilm wieder vereinigt, belüftet in eine Auffangwanne abgerieselt, weiterbelüftet und der Kammer wieder zugeführt wird;

c) nach der Füllung der Kammer erfolgt die weitere Oxidation der Gülle über den Stoffaustauschapparat bis zu ihrer vollständigen Absättigung;

d) die Sedimentation der Gülle läuft bei gleichzeitiger Oxidation ab;

e) die Entleerung der Kammer erfolgt nach der Sedimentation und dem Abschluß der Oxidation;

f) die phasenversetzte Füllung weiterer Kammern für eine separate Oxidation wird mittels des zugeordneten Stoffaustauschapparats und der Verfahrensschritte a - e vorgenommen;

g) die Phasenverschiebung beträgt jeweils eine Kammerfüllung.

Zweckmäßig sind die erste Kammer teilweise und die zweite Kammer vollständig gefüllt, wenn die dritte nach Abschluß eines Vorgangs entleert wird, wobei die Phasen um jeweils zwei technologische Stufen in den separaten Kammern versetzt sind.

Gemäß einer Ausgestaltung der Erfindung wird der Kreislauf über die Stoffaustauschapparate bis zur vollständigen Oxidation 13 mal für jede Kammer vorgenommen.

Im Verfahrensablauf wird vorteilhaft die Gülle einem Trennvorgang unterzogen, wobei die Feststoffe direkt zu einem Feststofflager gelangen und die Flüssigkeitsphase einer Kammer des Kompaktoxidationsbehälters zugeführt wird, in dem bei einem unteren Füllstand der Kammer bereits ein Umpumpen in einen Kreislauf vorgenommen wird, welcher über einen Stoffaustauschapparat erfolgt, bei dem ein Stoffaustausch in drei Stufen, einer ersten Stufe, einer Gegenstrom belüftung, indem die Flüssigkeit versprüht wird und mit einem vertikalen Luftgegenstromrieselfilm für eine zweite Stufe vereinigt, beim vertikalen Abrieseln in eine Auffangwanne läuft, dort in einer dritten Stufe wiederum mit dem Sauerstoff zum Austausch in Kontakt gebracht wird, um dann von der Auffangwanne in die Kammer zurückgelangend dem gleichen Kreislauf wieder zugeführt zu werden, der sich pro Kammer 12 bis 17 mal wiederholt, um in mehreren Kammern gleichzeitig ablaufend, jedoch phasenversetzt, vorgenommen zu werden, wobei die erste Kammer für einen Beginn eines Austauschvorgangs nur etwas gefüllt, die zweite Kammer gefüllt ist und die dritte Kammer gleichzeitig nach Abschluß eines Vorgangs entleert wird. Die behandelte Bio-Schlammphase kann dann erneut einer Biogasanlage zugeführt werden.

Vorteilhaft sind die Kammern für die Durchführung unterschiedlicher Vorgänge und Verfahrensschritte nacheinander und miteinander verbunden. Das Substrat wird bis zu einem Zehntel des Kammervolumens in einer Kammer angesammelt. Bis zu ihrer vollständigen Füllung wird weiter Substrat zugeführt, wobei das angesammelte und das neu zugeführte Substrat gleichzeitig der Oxidation zugeführt werden und das Substrat einer gleichzeitigen Sedimentation und Oxidation unterzogen wird. Der vorteilhafte Verfahrensverlauf ist gewahrt, wenn der Austauschvorgang in fünf technologischen Stufen sowie in drei gleiche Zeitanteile gegliedert ist und die Zeitanteile jeweils um zwei technologische Stufen versetzt beginnen, wobei die versetzten Stufen bei gleichen Zeitanteilen die technologischen Stufen eins und zwei sind.

Das Verfahren ist anwendungsgerecht, wenn die behandelten Abwasser einer weiteren Fest-Flüssig-Trennung zugeleitet und gemeinsam mit der Klarflüssigkeit aus dem Kompaktoxidationsbehälter einer weiteren Verwendung zugeführt werden.

Es ist ein Vorzug, daß der Kreislauf der Flüssigphase bis zur vollständigen Oxidation und $O_2$-Aufnahme in den Kammern je Kammer 13 mal vorgenommen wird und die Einführung der Flüssigphase in den Kreislauf des Stoffaustausches aus der Kammer bereits bei einem Zehntel des Kammervolumens beginnt. Vorteilhafterweise sollte eine Einrichtung, die bei der Durchführung des Verfahrens Verwendung findet, vor dem Kompaktoxidationsbehälter eine Fest-Flüssig-Trenneinrichtung vorgeordnet aufweisen. Jeder Kammer ist vorteilhaft ein Stoffaustauschapparat zugeordnet, der an seinem unteren Auslauf mit der Kammer verbunden, in den Kreislauf eingegliedert ist. Eine Rohrleitung befindet sich dabei an der Sohle der Kammer, um den Bioschlamm aufzunehmen, der wieder einer separaten Fest-Flüssig-Trennung zugeführt wird. Zur Realisierung des Verfahrensregimes sind Leitungen in unterschiedlicher Höhe aus der Stirnwand der Kammer austretend vorgesehen, wobei mit der Rohrleitung vorteilhaft der Bioschlamm am Kammerboden und das belüftete Substrat bzw. Abwasser mit der Rohrleitung in der mittleren Kammerhöhe einer Langzeitbiologie zugeführt wird.

Der Stoffaustauschapparat kann vorteilhaft ein Klein-Kühlturm sein, wobei der Klein-Kühlturm mit senkrechten Platten versehen ist, die über einem Auffangbecken angeordnet sind, wobei den Platten horizontal gegliedert Sprühdüsen zugeordnet sind, denen im Gegenstrom Luft zugeführt wird.

Das Verfahren weist mehrere Vorteile auf. Dem Kompaktoxidationsbecken sind mehrere Funktionen zugeordnet. Es kann vorteilhaft als eine Intensivkompaktoxidationsstufe bezeichnet werden, weil auf einer Grundfläche von nur 450 m² alle notwendigen Technologien mit den Vorrichtungen, wie Stoffaustauscher, Trenneinrichtung zur Fest-Flüssig-Trennung, Pumpen und verbindende Rohrleitungen mit entsprechenden Absperrvorrichtungen kompakt eine Einheit bildend als Intensivstufe integriert sind. Jede Kammer kann wahlweise einzeln oder gemeinsam durch entsprechende Einschiebungen oder gewählten Technologien die Funktion eines Oxidationsbeckens, eines Eindickerspeichers bzw. Vorlagebehälters übernehmen.

Durch die Kompaktheit der Funktionsteile der Einrichtung sinkt der Flächenbedarf entgegen einer mikrobiologischen Aufbereitungsanlage im herkömmlichen Sinne von 10 000 m² auf ein Zwanzigstel der genannten Fläche. Die zu planenden Investitionsmittel für die Bauausführung können auf ein Zehntel verringert werden, womit sich die erfinderische Lösung zur umfassenden Anwendung auch in kleineren Betrieben empfiehlt.

Der Energieaufwand verringert sich gegenüber dem vergleichbarer Verfahren unter Beachtung einer hohen Durchsatzleistung auf den fünften Teil des für herkömmliche Anlagen notwendigen Energiebedarfs. Die gesamte Einrichtung ist außerordentlich servicefreundlich, wartungsarm und wenig störanfällig. Der Einsatz von komplizierten Technologien wird vermieden.

Das erfindungsgemäße Verfahren tritt an die Stelle der bisher verwendeten Oxidationsverfahren von Abwasser und kann sowohl in Kombination mit Biogasanlagen als auch ohne diese zur Anwendung gelangen. Die Intensivkompaktoxidationsstufe ist vorteilhaft einer bestehenden Biogasanlage nachgeschaltet und garantiert somit einen hohen Effekt der Nitrifizierung und nahezu einen 95%igen $BSB_5$-Abbau. Somit schafft das Verfahren für die Abwasser-, Gülle- und Faulschlammbehandlung unter Nutzung der Abfallprodukte, wie Biogas und nährstoffreiche Feststoffe, günstige technische Voraussetzungen für die weitere Abwasserbehandlung innerhalb einer Langzeitbiologie bis zur Vorflutreife.

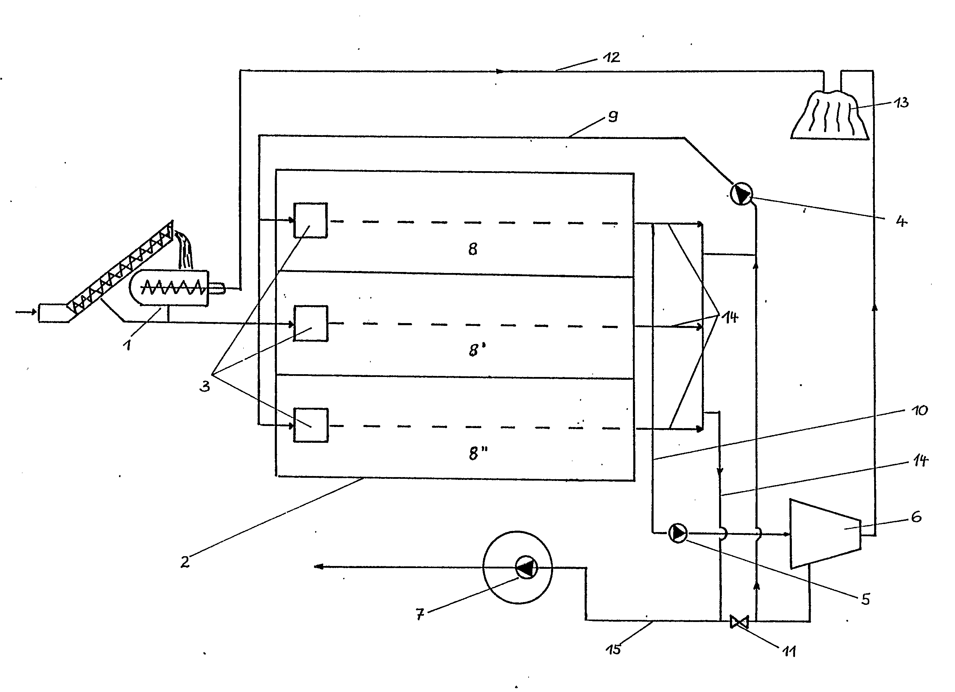

Die Erfindung wird im folgenden anhand eines in der Zeichnung schematisch dargestellten Ausführungsbeispiels im einzelnen erläutert.

Der dargestellten Einrichtung ist eine Fest-Flüssig-Trenneinrichtung vorgeschaltet, der die belasteten Abwasser, z. B. Gülle und Faulschlamm, zugeleitet werden. Die Trenneinrichtung kann aus einem Bürstensiebschneckenförderer 1 mit Presse, einem Dekanter oder aus einem Hydrozyklon mit Eindicker gebildet sein. Beim dargestellten Ausführungsbeispiel wird ein Bürstensiebschneckenförderer 1 als Trennaggregat verwendet, der einem Oxidationsbehälter 2 vorgeordnet ist. Der kompakt ausgeführte Oxidationsbehälter 2 ist in mehrere Abschnitte als Kammern 8 unterteilt. Im dargestellten Ausführungsbeispiel enthält der Kompaktoxidationsbehälter 2 zur Durchführung des Verfahrens drei Kammern 8; 8'; 8''. Der Kompaktoxidationsbehälter 2 ist nach oben unbedeckt und seine Kammern 8; 8'; 8'' sind untereinander gleichförmig ausgebildet. Auf jeder Kammer 8; 8'; 8'' ist ein Stoffaustauschapparat angeordnet, der vertikale Rieselfilmeinbauten aufweist. In dem verwendeten Verfahren wurde ein Klein-Kühlturm 3 zur Anwendung ausgewählt, der wesentliche Vorzüge aufweist. Zu den Kammern 8; 8'; 8'' und von den Kammern 8; 8'; 8'' wegführende Rohrleitungen sind mit Dosierpumpen 4 auf einer äußeren Rohrleitung 9 und einer Dosierpumpe 5 auf einer inneren Rohrleitung 10 ausgerüstet. Entsprechende Absperrventile 11 sind im Verfahren zur Durchflußregulierung vorgesehen. Von der Bürstensiebschneckenpresse 1 führen Fördermöglichkeiten, wie Förderbänder 12, zu einem Feststofflager 13. Die Rohrleitungen 9 münden verzweigt in die Stirnseiten der Kammern 8; 8'; 8''. Aus den Kammern 8; 8'; 8'' treten Abzugsrohre 14 aus, die in den einzelnen Kammern 8; 8'; 8'' in jeweils unterschiedlichen Höhen, bezogen auf den Kammerboden, angeordnet sind. Mit dem Feststofflager 13 verbunden ist ein Dekanter 6, der über eine Rohrleitung 10 mit der Dosierpumpe 5 und den Kammern 8; 8'; 8'' verbunden ist. Die Abzugsleitungen 14 und die Rohrleitung 9 sind im Bereich der Absperrventile 11 mit einer Leitung 15 verbunden, die über ein Pumpwerk 7 zu einer Langzeitbiologie führt. Das Verfahren wird mit der Einrichtung wie folgt durchgeführt:

Die belasteten Abwasser sind zur Schlammstabilisierung nach der vorherigen Fest-Flüssig-Trennung einer Oxidation zuzuführen, um mit Hilfe aerober Bakterien eine weitere Stufe der Abwasserbehandlung zu realisieren. Durch den Einsatz der Stoffaustauschapparate ist ein hoher Effekt bei der Oxidation der Flüssigphase möglich. Bei einem Füllstand einer Kammer 8; 8'; 8" von 50 m$^3$ entsprechend einem Zehntel des Kammervolumens wird der Oxidationsprozeß der jeweiligen Kammer durch das Öffnen eines Schiebers für eine Beschickungspumpe des Stoffaustauschapparates und Einschalten der Beschickungspumpe 4 begonnen. Das zu oxidierende Abwasser wird bis zur Beendigung der Oxidation über Rohrleitungen 9 im ständigen Kreislauf zwischen der Kompaktoxidationskammer 8; 8'; 8" über einen Grundablaß mit der Pumpe 4 zum Stoffaustauschapparat gepumpt.

Der als Stoffaustauschapparat verwendete Klein-Kühlturm 3 ist mit Sprühdüsen ausgerüstet, die horizontal über die Querschnittsfläche gleichmäßig verteilt sind. Dabei strömt vertikal im Gegenstrom Luft mit einem kontinuierlichen Druck gegen die sich abwärts bewegende Flüssigphase des Abwassers. Die aus den Sprühkegeln der Düsen gebildeten Flüssigkeitstropfen vereinen sich an der vertikal stehenden Austauschfläche zu einem Rieselfilm, der dabei durch eine Wellenform die fünf- bis zehnfache Oberfläche bildet und somit optimale Bedingungen für den Stoffaustausch schafft. Dieser Gegenluftstrom erzeugt also keine glatte, sondern eine stark wellige Oberfläche und trägt damit zur wesentlichen Verbesserung des Stoffaustausches bei. Er stellt die zweite Stufe dar, die Oxidation. Wenn das teilweise oxidierte Fugat diese Austauschflächen verläßt, tropft es in eine Auffangwanne unter dem Kühlturm 3 und kommt damit zum dritten Mal mit dem frischen Luftstrom in Berührung. Hiermit ist die dritte Stufe der Oxidation gebildet. Nach dem ersten Durchgang des teilweise oxidierten Fugats gelangt dieses in die Kammer 8; 8'; 8" zurück und wird erneut den beschriebenen Austauschweg über die Pumpe 4, den Stoffaustauschapparat 3 zurück in die Kammer, nehmen.

Die Kammern 8; 8'; 8" werden ohne Unterbrechung phasenversetzt gefüllt und wieder entleert. Während in der ersten Kammer 8 der Oxidationsvorgang beginnt, ist die zweite Kammer 8' vollständig gefüllt und die dritte Kammer 8" wird geleert. Die Oxidationszeit richtet sich nach der anfallenden Abwasser- bzw. Substratmenge und der Belastung des Substrats. Die Verweilzeit im Oxidationskreislauf des aufzubereitenden Substrats als Faulschlamm kann bis zu 40 Stunden betragen, der Zulauf beträgt 12 m$^3$ pro Stunde. Bei einem gewählten Pumpendurchsatz von 100$^3$/h wird der Kammerinhalt von 500 m$^3$ 13 mal total dem Stoffaustauschapparat zur Oxidation zugeführt.

Der Verfahrensverlauf gliedert sich in drei gleiche Zeitanteile und fünf unterschiedliche technologische Stufen.

Die Kammern und die drei Stoffaustauschapparate haben im Intervall der technologischen Stufen und in Abhängigkeit von der Beschickungszeit miteinander verbundene Prozeßanforderungen zu erfüllen, die zum Teil gleichzeitig zeitlich versetzt und einzeln wirksam sind.

### 1. Stufe

Ansammlung des zu behandelnden Substrats, bis ca. 1/10 einer Kammer gefüllt ist.

### 2. Stufe

Oxidation des zulaufenden frischen und des in der Kammer angesammelten Substrats.

### 3. Stufe

Nach beendigung der Füllung einer Kammer erfolgt die weitere Oxidation des Substrats durch ständiges Umpumpen über den Stoffaustauschapparat.

### 4. Stufe

Sedimentation des biologischen Schlammes bei gleichzeitiger Oxidation.

### 5. Stufe

Entleerung der Kammer nach der Sedimentation des biologischen Schlammes und der Oxidation, Behandlung mit nachfolgender mechanischer Trennung in Klarflüssigkeit und biologischem Schlammfeststoff.

Sind die Stufen 1 und 2 für die Kammer 1 erreicht, wird die Kammer 2 in der Folge des verfahrenstechnischen Regimes nach den Stufen 1 und 2 genutzt usw. Diese Phasenverschiebung der einzelnen verfahrenstechnischen Operationen ist in einer Zyklustabelle dargestellt. Die Zeitdauer für die verfahrenstechnischen Operationen beträgt je Kammer

| Stufen 1 und 2 | 1/3 der Gesamtverweilzeit | in |
|---|---|---|
| Stufen 3 und 4 | 1/3 " " | der |
| Stufe 5 | 1/3 " " | Kompaktoxidationsanlage. |

Während der Stufe 5 ist der Stoffaustauschapparat außer Betrieb.

| Zyklustabelle | | | | | | | | | |
|---|---|---|---|---|---|---|---|---|---|
| Kammer 1 | Stufe | 1;2 | 3;4 | 5 | 1;2 | 3;4 | 5 | 1;2 | 3;4 |
| Kammer 2 | Stufe | - | 1;2 | 3;4 | 5 | 1;2 | 3;4 | 5 | 1;2 |
| Kammer 3 | Stufe | - | - | 1;2 | 3;4 | 5 | 1;2 | 3;4 | 5 |

Um ein kontinuierliches Befüllen, Oxidieren und Entleeren der aufzubereitenden Substrate entsprechend den Ausgangswerten zu erreichen, ist im Beispiel eine Dreikammeranlage mit drei Stoffaustauschapparaten ausgewählt worden. Aus der so behandelten Flüssigkeit bildet sich infolge der Vermehrung der Bakterien ein nährstoffreicher Bioschlamm sowie ein stickstoffreduziertes Abwasser. Dieser bei der Intensivoxidation anfallende Bioschlamm kann zur Biogasproduktion bzw. über den Dekanter 6 zur Feststoffgewinnung genutzt werden. Das anfallende Fugat und die Klärflüssigkeit aus der Intensivoxidation wird in Abhängigkeit von der Sedimentation im Kompaktoxidationsbehälter 2 stufenweise mit dem füllstandsabhängigen Pumpwerk 7 einer weiteren mikrobiologischen Behandlung in Teichanlagen der Langzeitbiologie durchgeführt. Bei Bedarf kann dieses aufbereitete Wasser auch zu Beregnungszwecken für landwirtschaftliche Kulturen zum Einsatz gelangen.

## Ansprüche

1. Verfahren zur Aufbereitung von Gülle oder Abwasser, bei dem die Gülle während des Oxidationsvorgangs eine erste Kammer füllend und sich phasenversetzt in weiteren Kammern fortsetzend einer Gegenstrombelüftung ausgesetzt bei laufender Oxidation mehrfach versprüht und wieder vereinigt, belüftet und bei gleichzeitiger Sedimentation bis zur vollständigen Absättigung der Gülle behandelt wird.

2. Verfahren nach Anspruch 1, bei dem die Gülle, die in einer Biogasanlage behandelt wurde, in einem Oxidationsvorgang partiell einer Gegenstrombelüftung ausgesetzt und dann einer Kammer zugeführt wird, **dadurch gekennzeichnet,**

a) daß von drei zusammengehaltenen Kammern mit jeweils zugeordnetem Stoffaustauschapparat in Form eines Klein-Kühlturms die erste Kammer bis zu einem Zehntel des Kammervolumens gefüllt wird;

b) Oxidation der in der ersten Kammer vorhandenen und der weiter zulaufenden Gülle über den Stoffaustauschapparat, mit dem die Gülle versprüht und in einem Rieselfilm wieder vereinigt, belüftet, in eine Auffangwanne abgerieselt weiterbelüftet und der Kammer wieder zugeführt wird;

c) nach Füllung der Kammer weitere Oxidation der Gülle über den Stoffaustauschapparat bis zu ihrer vollständigen Absättigung;

d) Sedimentation bei gleichzeitiger Oxidation;

e) Entleerung der Kammer nach der Sedimentation und Abschluß der Oxidation;

f) phasenversetzte Füllung der weiteren Kammer für eine separate Oxidation mittels des jeweils zugeordneten Stoffaustauschapparates und Vornahme der Verfahrensschritte a bis e;

g) Ablauf der Phasenverschiebung um jeweils eine Kammerfüllung

3. Verfahren nach den Ansprüchen 1 und 2,

dadurch gekennzeichnet,
daß die erste Kammer teilweise gefüllt und die zweite Kammer gefüllt ist, wenn die dritte nach Abschluß eines Vorgangs entleert wird.

4. Verfahren nach den Ansprüchen 1 und 3,
dadurch gekennzeichnet,
daß die Phasen um jeweils zwei technologische Stufen in den separaten Kammern versetzt sind.

5. Verfahren nach den Ansprüchen 1 und 3,
dadurch gekennzeichnet,
daß der Kreislauf über die Stoffaustauschapparate bis zur vollständigen Oxidation 13 mal für jede Kammer vorgenommen wird.

6. Einrichtung zur Durchführung des Verfahrens nach den Ansprüchen 1 bis 5,
dadurch gekennzeichnet,
daß mehreren Kammern (8; 8'; 8") ein Stoffaustauschapparat (3) zugeordnet ist, der mit den Kammern (8; 8'; 8") in Verbindung in den Kreislauf eingebunden ist.

7. Einrichtung nach Anspruch 6,
dadurch gekennzeichnet,
daß der Kompaktoxidationsbehälter (2) drei Kammern (8; 8'; 8") aufweist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A- 567 241 (V. LAMY)<br>* Seite 1, Zeilen 4-10; Seiten 2,3, Zusammenfassung *<br>--- | 1-4 | C 02 F 3/12<br>C 02 F 3/04<br>C 02 F 3/24 |
| A | FR-A-2 550 522 (SA VOR)<br>* Seite 3, Zeile 32 - Seite 4, Zeile 19 *<br>--- | 1-4,7 | |
| A | DE-A-3 147 920 (LINDE AG)<br>* Seite 7, Zeile 22 - Seite 9, Zeile 24; Figur 2 *<br>--- | 1,2,7 | |
| A | US-A-4 267 130 (H.D. CURTIS)<br>* Spalte 2, Zeilen 63-68 *<br>--- | 2 | |
| A | CH-A- 668 252 (E. WALTHER)<br>* Zusammenfassung *<br>----- | 1,2 | |

|  |  |  | **RECHERCHIERTE SACHGEBIETE (Int. Cl.5)** |
|---|---|---|---|
| | | | C 02 F<br>A 01 C |

Der vorliegende Recherchenbericht wurde für alle Patentansprüche erstellt

| Recherchenort | Abschlußdatum der Recherche | Prüfer |
|---|---|---|
| DEN HAAG | 16-11-1989 | GONZALEZ Y ARIAS M.L. |